(19) Europäisches Patentamt
European Patent Office
Office européen des brevets

(11) Veröffentlichungsnummer: **0 247 484**
**B1**

(12) **EUROPÄISCHE PATENTSCHRIFT**

(45) Veröffentlichungstag der Patentschrift: **16.08.90**

(21) Anmeldenummer: **87107164.3**

(22) Anmeldetag: **18.05.87**

(51) Int. Cl.[5]: **A 01 N 29/02**, C 07 C 21/18, C 07 C 17/26, C 07 C 21/19, C 07 C 21/22, C 07 C 21/215

# (54) Halogenolefine.

(30) Priorität: **30.05.86 DE 3618115**

(43) Veröffentlichungstag der Anmeldung:
**02.12.87 Patentblatt 87/49**

(45) Bekanntmachung des Hinweises auf die Patenterteilung:
**16.08.90 Patentblatt 90/33**

(84) Benannte Vertragsstaaten:
**AT BE CH DE FR GB IT LI NL**

(56) Entgegenhaltungen:
**CHEMISTRY LETTERS, 1979, Seiten 983-986, The Chemical Society of Japan, Tokio, JP; S.-I. HAYASHI et al.: "Convenient procedures for conversion of carbonyl compounds to gem-difluoroolefins and their selective reductions to monofluoroolefins"**

(73) Patentinhaber: **BAYER AG**
**D-5090 Leverkusen 1 Bayerwerk (DE)**

(72) Erfinder: **Naumann, Klaus, Dr.**
**Richard-Wagner-Strasse 9**
**D-5090 Leverkusen 1 (DE)**
Erfinder: **Becker, Benedikt, Dr.**
**Metzkausener Strasse 14**
**D-4020 Mettmann (DE)**
Erfinder: **Behrenz, Wolfgang, Dr.**
**Untergründemich 14**
**D-5063 Overath (DE)**
Erfinder: **Homeyer, Bernhard, Dr.**
**Obere Strasse 28**
**D-5090 Leverkusen 3 (DE)**
Erfinder: **Stendel, Wilhelm, Dr.**
**In Den Birken 55**
**D-5600 Wuppertal 1 (DE)**

(56) Entgegenhaltungen:
**CHEMICAL ABSTRACTS, Band 98, Nr. 5, 31. Januar 1983, Seite 624, Zusammenfassung Nr. 34296q, Columbus, Ohio, US; & JP-A-57 142 926 (SAGAMI CHEMICAL RESEARCH CENTER) 03-09-1982, & Chemical Abstracts, vol. 98, Januar-Juni 1983, Formula Index, A-C15, Seite 1041F, Absatz C12H22F2 (Kat. L), American Chemical Society, Columbus, Ohio, US**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 247 484 B1

Courier Press, Leamington Spa, England.

(56) Entgegenhaltungen:

CHEMICAL ABSTRACTS SERVICE REGISTRY HANDBOOK, Number Section, 1978 supplement, registry numbers 65229-06-3 through 67087-27-8, Seiten 803RG,819RG,870RG,882RG,946RG,1029RG,1303RG, American Chemical Society, Columbus, Ohio, US

PESTICIDE SCIENCE, Band 17, August 1986, Seiten 441-448, Barking, GB; G.G. BRIGGS et al.: "Some fluorine-containing pheromone analogues"

**Beschreibung**

Die Erfindung betrifft die Verwendung von langkettigen Halogenolefinen als arthropodizide (vorzugsweise insektizide) und nematizide Schädlingsbekämpfungsmittel, die diese Halogenolefine als arthropodizide und nematizide Wirkstoffe enthalten.

Es ist bekannt, daß bestimmte ungesättigte langkettige Carbonsäureester, wie z.B. (E,E)-11-Methoxy-3,7,1-trimethyl-2,4-dodecandiensäure-isopropylester als Schädlingsbekämpfungsmittel eingesetzt werden können (Chem. Engng. News *49,* Nr. 29, S. 33—34). Obwohl langkettige Halogenolefine zum Teil beschrieben sind (verg. z.B. C.A. *66,* 120597; C.A. *85,* 77532n; C.A. *91,* 210992e und C.A. *98,* 89460u), ist ihre mögliche Verwendung zur Abtötung von Arthropoden und Nematoden bisher nicht bekannt geworden.

Es wurde nun gefunden, daß die langkettige Halogenolefine der Formel (I)

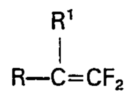

(I)

in welcher

R für einen geradkettigen oder verzweigten Alkylrest mit 8 bis 25 Kohlenstoffatomen in der geraden Kette steht, wobei der Alkylrest gegebenenfalls einfach oder mehrfach durch eine —C≡C— und/oder

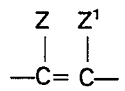 Gruppierung unterbrochen ist,

in welcher

Z und $Z^1$ gleich oder verschieden sind und für Wasserstoff oder Methyl stehen und

$R^1$ für Wasserstoff, Alkyl oder Halogen steht,

als Arthropodizide (vorzugsweise Insektizide) und Nematizide verwendet werden können.

Die langkettigen Halogenolefine der Formel (I) können erhalten werden, indem man

(a) Triphenylphosphoniumsalze der Formel (II)

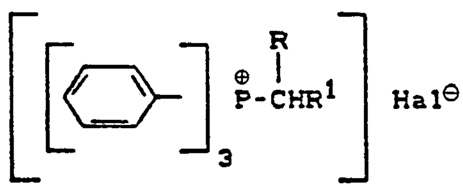

(II)

in welcher

R und $R^1$ die oben bei der Formel (I) angegebenen Bedeutungen haben und

Hal für Halogen steht,

mit Chlordifluormethan, in Gegenwart von $C_1$—$C_4$-Alkyllithium-Verbindungen in Gegenwart von inerten Verdunungsmitteln und in einer Schutzgasatmosphäre umsetzt,

oder

(b) Carbonyl-Derivate der Formel (III)

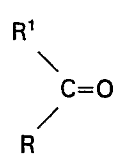

(III)

in welcher

R und $R^1$ die oben bei Formel (I) angegebenen Bedeutungen haben,

mit Difluormethanen der Formel (IV)

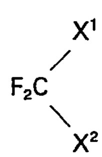

(IV)

in welcher

$X^1$ für Brom steht und

$X^2$ für Chlor oder Brom steht,

und mit Phosphinen der Formel (V)

$$PQ_3 \qquad (V)$$

in welcher

Q für Phenyl oer Dimethylamino steht,

in Gegenwart von inerten Verdünnungsmitteln und in einer Schutzgasatmosphäre umsetzt (vgl. THL *12,* 895—898 (1976); Chem. Lett. *1979,* 983—986 und THL *23,* 2323—2326 (1982)).

Die Verbindungen der Formel (I) verfügen über Eigenschaften, die ihre Verwendung als arthropodizide und nematizide Wirkstoffe ermöglicht.

In den allgemeinen Formeln steht R vorzugsweise für geradkettiges Alkyl mit 8 bis 25, vorzugsweise 10 bis 22, insbesondere 12 bis 20 Kohlenstoffatomen. Der Alkylrest kann ein oder mehrfach, vorzugsweise 1- bis 6-fach, insbesondere 1- bis 4-fach und besonders bevorzugt 1- oder 2-fach durch die —C≡C-Gruppe und oder die —C(Z)=C($Z^1$)-Gruppe (vorzugsweise nur durch die —C(Z)=C($Z^1$)-Gruppe) unterbrochen sein, wobei Z und $Z^1$ vorzugsweise gleich sind und für Wasserstoff oder Methyl stehen. Besonders bevorzugt wird der Alkylrest R, welcher nicht durch ungesättigte Gruppen unterbrochen ist. Der Rest $R^2$ hat die gleiche vorzugsweise Bedeutung wie der Rest R, wobei jedoch Alkyl 12 bis 25, vorzugsweise 12 bis 22, insbesondere 14 bis 20 Kohlenstoffatome enthält.

Alkyl $R^1$ is geradkettig oder verzweigt und enthält vorzugsweise 1 bis 6, insbesondere 1 bis 4 Kohlenstoffatome, wobei beispielhaft Methyl, Ethyl, n-Propyl, i-Propyl, n-, i.-, s.- und t.-Butyl (vorzugsweise Methyl und Ethyl) genannt seien.

Halgen $R^1$ bedeutet Fluor, Chlor, Brom und Iod, vorzugsweise Fluor, Chlor und Brom, insbesondere Fluor oder Chlor.

In der allgemeinen Formeln steht $R^1$ besonders bevorzugt für Wasserstoff, Methyl oder Chlor, insbesondere für Wasserstoff.

Bevorzugt werden erfindungsgemäß die Verbindungen der Formel (I) verwendet, in welcher

R für einen geradkettigen oder verzweigten Alkylrest mit 10 bis 22 Kohlenstoffatomen in der geraden Kette steht, wobei der Alkylrest gegebenenfalls einfach bis sechsfach durch eine —C≡C— und/oder

Z $Z^1$
| |
—C= C— Gruppierung unterbrochen ist,

in welcher

Z und $Z^1$ gleich oder verschieden sind und für Wasserstoff oder Methyl stehen und

$R^1$ für Wasserstoff, Alkyl mit 1 bis 6 Kohlenstoffatomen oder für Fluor, Chlor oder Brom steht.

Besonders bevorzugt werden die Verbindungen der Formel (I) verwendet, in denen

R für einen geradkettigen oder verzweigten Alkylrest mit 12 bis 20 Kohlenstoffatomen in gerader Kette steht, wobei der Alkylrest gegebenenfalls einfach bis vierfach durch

Z $Z^1$
| |
eine —C= C— Gruppierung unterbrochen ist,

in welcher

Z und $Z^1$ gleich oder verschieden sind und für Wasserstoff oder Methyl stehen und

$R^1$ für Wasserstoff, Methyl, Ethyl, n-Propyl, i-Propyl, Fluor oder Chlor steht.

Ganz besonders bevorzugt werden diejenigen Verbindungen der Formel (I) verwendet, in denen

R die oben bei Formel (I) angegebenen besonders bevorzugten Bedeutungen hat und

$R^1$ Wasserstoff steht.

Verwendet man nach Verfahrensvariante (a) Pentadecyl-triphenylphosphoniumbromid, Chlordifluormethan und Methyllithium als Ausgangsstoffe, so kann der Reaktionsablauf durch das folgende Formelschema wiedergegeben werden:

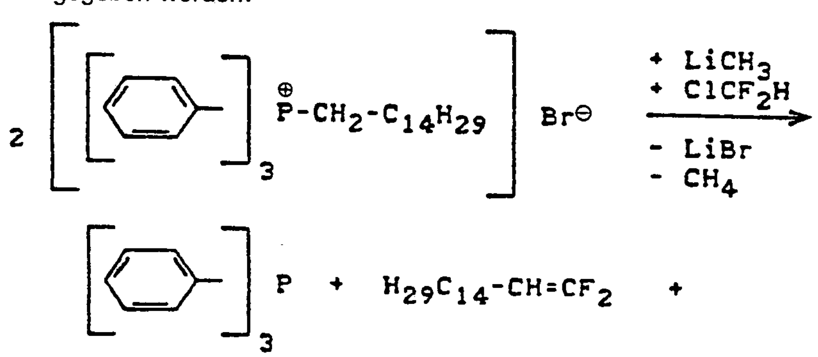

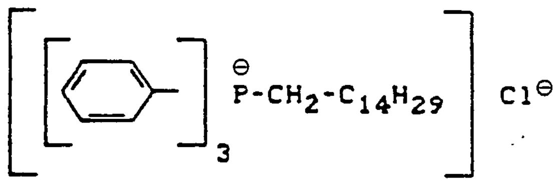

Verwendet man nach Verfahrensvariante (b) Bromchlordifluormethan, Pentadecanal und Tri(dimethylamino)phosphin als Ausgangsstoffe, so kann der Reaktionsablauf durch das folgende Formelschema wiedergegeben werden:

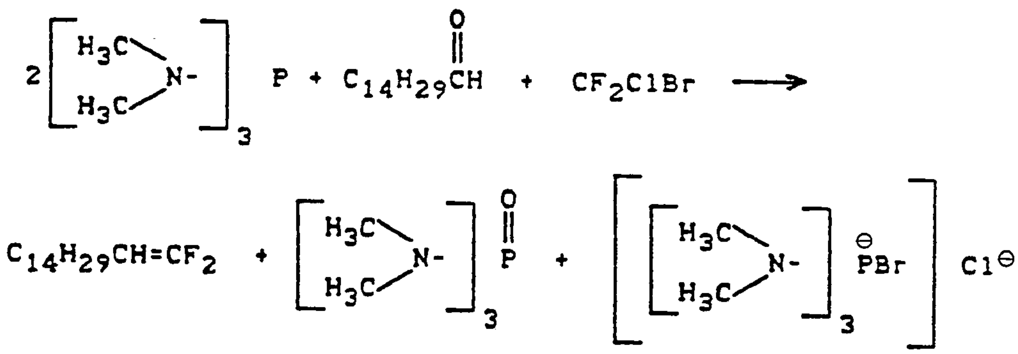

Die beim Verfahren (a) als Ausgangsstoffe zu verwendenden Triphenylphosphoniumsalze sind durch die Formel (II) allgemein definiert.

Als Beispiele für die Verbindungen der Formel seien genannt:

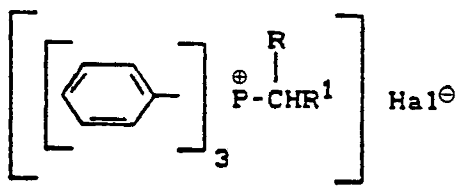

(II)

Hal = Chlor oder Brom

TABELLE 1

| $R^1$ | R |
|---|---|
| H | $-C_{12}H_{25}$-n |
| H | $-C_{13}H_{27}$-n |
| H | $-C_{14}H_{29}$-n |
| H | $-C_{15}H_{31}$-n |
| H | $-C_{16}H_{33}$-n |
| H | $-C_{17}H_{35}$-n |
| H | $-C_{18}H_{37}$-n |
| H | $-C_{19}H_{39}$-n |
| H | $-C_{20}H_{41}$-n |
| H | $-C_{21}H_{43}$-n |
| H | 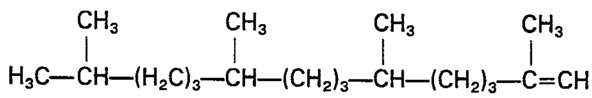 |
| H | $H_3C-(CH_2)_7-CH=CH-(CH_2)_6-CH_2-$ |
| H | $H_3C-(CH_2)_4-CH=CH-CH_2-CH=CH-(CH_2)_6-CH_2-$ |
| H | $H_3C-(CH_2)_7-CH=CH-(CH_2)_6-CH_2$ |

Die Verbindungen der Formel (II) sind bekannt und/oder lassen sich auf einfache Weise nach bekannten Methoden herstellen (vergl. z.B. DE—OS 25 51 914; J. Org. Chem. *48,* 917—927 und Chem. Ber. *116,* 3264—3266).

Die beim Verfahren (b) als Ausgangsstoffe zu verwendenden Carbonyl-Derivate sind durch die Formel (III) allgemein definiert.

Als Beispiele für die Verbindungen der Formel (III) seien genannt:

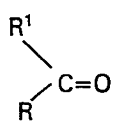

(III)

TABELLE 2

| $R^1$ | R |
|---|---|
| H | $-C_{12}-H_{25}$-n |
| H | $-C_{13}-H_{27}$-n |
| H | $-C_{14}-H_{29}$-n |
| H | $-C_{15}-H_{31}$-n |
| H | $-C_{16}-H_{33}$-n |
| H | $-C_{17}H_{35}$-n |
| H | $-C_{19}H_{39}$-n |
| H | $-C_{20}H_{41}$-n |
| H | $-(H_2C)_8-CH=CH-(CH_2)_6-CH_3$ |
| H | $-(H_2C)_8-CH=CH-CH_2-CH=CH-(CH_2)_4-CH_3$ |
| $CH_3$ | $-C_{12}H_{25}$-n |
| $CH_3$ | $-C_{15}H_{31}$-n |

Die Carbonyl-Derivate der Formel (III) sind bekannt oder lassen sich auf allgemein bekannte Art und Weise durch Ozonisierung von bekannten langkettigen terminalen Olefinen bzw. durch Oxidation von langkettigen Alkoholen herstellen.

Die beim Verfahren (b) außerdem als Ausgangsstoffe zu verwendenden Difluormethane sind durch die Formel (IV) allgemein definiert. In dieser Formel (IV) stehen $X^1$ für Brom und $X^2$ für Chlor oder Brom.

Als Beispiele für die Verbindungen der Formel (IV) seien genannt:

Dibromfluormethan und Bromchlordifluormethan.

Die Verbindungen der Formel (IV) sind bekannt.

Die beim Verfahren (b) weiterhin als Ausgangsstoffe zu verwendenden Phosphine sind durch die Formel (V) allgemein definiert. In dieser Formel steht Q für Phenyl oder Dimethylamino.

Als Beispiele für die Phosphine der Formel (V) seien genannt:

Triphenylphosphin und Tris-(dimethylamino)-phosphin.

Die Phosphine der Formel (V) sind bekannte Verbindungen der organischen Chemie.

Das Verfahren (a) zur Herstellung der neuen langkettigen Halogenolefine der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise Ether wie Diethyl- und Dibutylether, Glykoldimethylether und Diglykoldimethylether, Tetrahydrofuran und Dioxan.

Als Alkyllithium-Verbindungen können bei dem Verfahren (a) alle üblicherweise für derartige Umsetzungen verwendbaren Alkyllithium-Verbindungen eingesetzt werden. Vorzugsweise in Frage kommen: Methyl- und n-Butyllithium.

Die Reaktionstemperaturen können bei dem Verfahren (a) in einem größeren Bereich variiert werden. Im allgemeinen arbeitet man bei Temperaturen zwischen −40°C und +60°C, vorzugsweise bei Temperaturen zwischen −20°C und +30°C.

Das Verfahren (a) wird in allgemeinen unter Normaldruck durchgeführt. Es ist jedoch auch möglich, unter erhöhtem oder vermindertem Druck zu arbeiten.

Bei der Durchführung das Verfahrens (a) setzt man auf 1 Mol Triphenylphosphoniumsalz der Formel (II) 1,5 bis 3,0 Mol, vorzugsweise 2,0 bis 2,6 Mol Alkyllithium-Verbindung und 1,5 bis 3,0 Mol, vorzugsweise 2,0 bis 2,6 Mol Chlordifluormethan ein. Die Reaktion wird in einer Inertgasatmosphäre durchgeführt. Als Inertgas wird bevorzugt Stickstoff verwendet. Die Aufarbeitung geschieht nach üblichen Methoden.

Das Verfahren (b) zur Herstellung der langkettigen Halogenolefine der Formel (I) wird vorzugsweise unter Verwendung von Verdünnungsmitteln durchgeführt. Als Verdünnungsmittel kommen vorzugsweise die Lösungsmittel in Frage, die bereits für das Verfahren (a) angegeben worden sind.

Die Reaktionstemperaturen können bei dem Verfahren (b) in einem größeren Bereich variiert werden. Im allgemeinen arbeitet man bei Temperaturen zwischen −10°C und +60°C, vorzugsweise bei Temperaturen zwischen 0°C und +30°C.

Das Verfahren (b) wird in allgemeinen unter Normaldruck durchgeführt. Es ist jedoch auch möglich, unter erhöhtem oder vermindertem Druck zu arbeiten. Vorzugsweise wird ein Inertgas (zweckmäßigerweise Stickstoff) eingesetzt.

Bei der Durchführung des Verfahrens (b) setzt man auf 1 Mol Carbonyl-Derivat der Formel (III) 1 bis 3,0 Mol vorzugsweise 2 bis 2,6 Mol Difluormethan der Formel (IV) und 2 bis 6 Mol, vorzugsweise 4 bis 5 Mol Phosphin der Formel (V) ein. Die Aufarbeitung geschieht nach Üblichen Methoden.

Die Schädlingsbekämpfungsmittel eignen sich zur Bekämpfung von tierischen Schädlingen, vorzugsweise Arthropoden und Nematoden, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgate, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella malculipennis, Malacosoma neustria, Euproctis chrysorrhoea, Lymantria spp. Bucculatrix thurberiella, Phyllocnistic citrella, Agrotis spp., Euxoa spp., Feltia spp., Earias insulana, Heliothis spp., Spodoptera exigua, Mamestra brassocae, Panolis flammea, Prodenia litura, Spodoptera spp., Trichoplusia ni, Carpocapsa pomonella, Pieris spp., Chilo spp., Pyrausta nubilalis, Ephestia keuhniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopetha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Orzyaephilus surinamensis, Anthonomus spp., Sitophilus spp., Otiorrhynchus sulcatus, Cosmopolites sordidus, Ceuthorrhynchus assimilis, Hypera postica, Dermestes spp. Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lassius spp., Monomorium pharaonis, Vespa spp.

Aus der Ordnung der Diptera z.B. Aedes spp., Anoppheles spp., Culex spp., Drosophila melanogaster, Musca spp., Fannia spp., Calliphora arythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phobia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Verbindungen der Formel (I) eignen sich besonders gut zur Bekämptfung von pflanzenschädigenden Insekten wie z.B. Plutella maculipennis und Spodoptera frugiperda, und Nematoden wie Meloidogyne incognita. Weiterhin können sie eingesetzt werden bei der Bekämpfung von Bodeninsekten, wie z.B. Phorbia-antiqua Maden und Diabrotica balteata. Sie eignen sich außerdem als Entwicklungshemmer beim Einsatz gegen z.B. Fliegenmaden. Die Verbindungen der Formel (I) bzw. (Ia) werden vorzugsweise im Pflanzenschutz, Vorratsschutz, Materialschutz, sowie zur Bekämpfung lästiger Insekten in Haushalt und im gewerblichen Bereich eingestzt (insbesondere im Pflanzenschutz und Vorratsschutz).

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stofen und in Hüllmassen für Saatgut, ferner in Formulierungen mit Brennsätzen, wie Rächerpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von overflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnapthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionnen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamide und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage; z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: a.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molbdän und Zink verwendet werden.

Die erfindungsgemäßen Formulierungen enthalten neben wenigstens einem Wirkstoff der Formel (I) und wenigstens einem Streckmittel oder Trägerstoff zusätzlich wenigstens ein Overflächenaktives Mittel.

Die Formulierungen enthalten im allgemeinen zwischen 0,1 und 95 Gewichtsprozent Wirkstoff, vorzugsweise zwischen 0,5 und 90%.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die Wirkstoffe der Formeln (I) und (Ia) eignen sich auch zur Bekämpfung von Arthropoden, wie Insekten, Milben oder Zecken, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sog. Versuchstiere, wie z.B. Hamster, Meerschweinchen, Ratten und Mäuse befallen.

Durch die Bekämpfung dieser Arthropoden sollen insbesondere Leistungsminderungen (bei Fleisch, Milch, Wolle, Häuten, Eiern, Honig usw.) vermindert werden, so daß durch den Einsatz des Wirkstoffs eine wirtschaftliche und einfache Tierhaltung möglich ist.

Die Anwendung der erfindungsgemäß verwendbaren Wirkstoffe geschieht in diesen Bereichen in bekannter Weise durch dermale Anwendung in Form beispielsweise des Tauchens oder badens (Dippen), Sprühen (Spray), Aufgießens (Pour-on und Spot-on), des Waschens, des Einpuders sowie it Hilfe von

wirkstoffhaltigen Formkörpern, wie Halsbändern, Ohrmarken, Schwanzmarken, Gliedmaßenbändern, Halftern, Markierungsvorrichtungen usw.

Die Wirkstoffe eigen sich besonders zur Bekämpfung von Ektoparasiten wie z.B. Psoroptes ovis und Boophilus microplus.

Die Herstellung der Wirkstoffe soll anhand der folgenden Beispiele erläutert werden.

Herstellungsbeispiele

Beispiel (Ia—1)

$$CH_3—(CH_2)_{13}—CH=CF_2$$

(Verfahren (a))

Zu einer Suspension von 240 g (0,43 Mol) Pentadecyl-triphenyl-phosphoniumbromid in 450 ml Tetrahydrofuran wird in einer Stickstoffatmosphäre 190 ml einer 23%igen Losung (0,47 Mol) von Butyllithium in n-Hexan bei 0°C zugetropft und 30 Min. heftig gerührt. Anschließend werden 121 g einer 34%igen Lösung (0,47 Mol) von Chlordifluormethan in Tetrahydrofuran bei 20°C zugetropft, 8 Stunden gerührt und dann noch einmal mit den gleichen Mengen Butyllithium und Chlordifluormethan vesetzt. Das Reaktionsgemisch wird ca. 15 Stunden bei 20°C gerührt. Das entstandene Triphenylphosphin wird mit überschüssigem Methyliodid in der Reaktionsmischung umgesetzt. Das gebildete Salz wird abfiltriert, das Filtrat eingeengt, in Petrolether aufgenommen und im Vakuum destilliert.

Man erhält so 89 g (80% der Theorie) 1,1-Difluor-1-hexadecen vom Siedepunkt Kp: 90—95°C/40 -a (0,4 mbar).

Beispiel (Ia—2)

$$CH_3—(CH_2)_{15}—CH=CF_2$$

(Verfahren (b))

Zu einer Lösung von 50,8 g (0,2 Mol) Heptadecanal und 73 g (0,44 Mol) Bromchlordifluormethan in 500 ml Tetrahydrofuran werden in einer Stickstoffatmosphäre bei 10°C eine Lösung von 143 g (0,88 Mol) Tris-(dimethylamino)-phosphin zugegeben. Nach einer Stunde wird eine Mischung von 27 g (1,5 Mol) Wasser und 48 g (1,5 Mol) Methanol bei einer Temperatur von 0°C zugetropft und die wäßrige Phase abgetrennt. Die organische Phase wird mit Sodalösung, verdünnter Salzsäure und Wasser gewaschen. Anschließend wird die organische Phase getrocknet und eingeengt. Der Rückstand wird in Vakuum rektifiziert.

Man erhält so 37,5 g (65% der Theorie) 1,1-Difluor-1-octadecen vom Siedepunkt Kp: 99—100°C/10 Pa (0,1 mbar).

Analog Verfahren (a) und (b) bzw. Beispiel (Ia—1) und (Ia—2) können die folgenden Verbindungen der Formel (Ia) hergestellt werden:

$$R^2—C(R^1)=CF_2 \qquad (Ia)$$

TABELLE 3

| Bsp.-Nr. | $R^1$ | $R^2$ | Physikal. Konstante |
|---|---|---|---|
| Ia—3 | H | $-C_{17}H_{35}$-n | Kp: 82—83°C/20 Pa (0,2 mbar) |
| Ia—4 | H | $-C_{13}H_{27}$-n | Kp: 59—60°C/25 Pa (0,25 bar) |
| Ia—5 | H | $-C_{15}H_{31}$-n | Kp: 95°C/10 Pa (0,1 mbar) |
| Ia—6 | H | $-(CH_2)_7-CH=CH-(CH_2)_7-CH_3$ | Kp: 100°C/10Pa (0,1 mbar) |
| Ia—7 | H | $-(CH_2)_7-CH=CH-CH_2-CH=CH-(CH_2)_4-CH_3$ | Kp: 100°C/10 Pa (0,1 mbar) |
| Ia—8 | H | $-C_{19}H_{39}$-n | Kp: 130—133°C/10 Pa (0,1 mbar) |
| Ia—9 | H | $-C_{12}H_{25}$-n | Kp: 55—56°C/25 Pa (0,25 mbar) |
| Ia—10 | H | $-C_{21}H_{43}$-n | Kp: 140—141°C/10 Pa (0,1 mbar) |
| Ia—11 | H | $-C_{18}H_{37}$-n | Kp: 118°C/8 Pa (0,08 mbar) |
| Ia—12 | H | 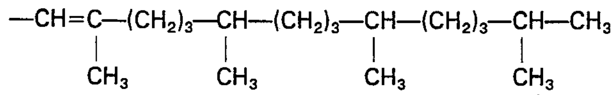 | Kp: 115—120°C/150 Pa (0,5 mbar) |

Die Verbindungen der Formel (I) können analog den oben angegebenen Verfahren (a) und (b) hergestellt werden bzw. lassen sie sich nach den Verfahren herstellen, die aus der Literatur bekannt sind (vergl. THL *12,* 895—898 (1976); Chem. Lett. *1979,* 983—986 und THL *23,* 2323—2326 (1982)):

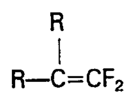 (I)

TABELLE 4

| Bsp.-Nr. | $R^1$ | $R^2$ | Physikal. Konstante |
|---|---|---|---|
| I—1 | H | $-C_9H_{19}$-n | Kp: 28°C/60 Pa (0,6 mbar) |
| I—2 | H | $-C_{11}H_{23}$-n | Kp: 63°C/60 Pa (0,6 mbar) |
| I—3 | H | $-C_{10}H_{21}$-n | Kp: 56°C/60 Pa (0,6 mbar) |
| I—4 | H | 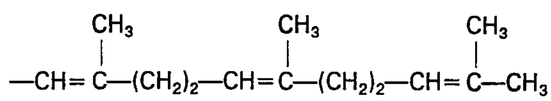 | KP: 105°C/40 Pa (0,4 mbar) |
| I—5 | $CH_3$ | $-C_{10}H_{21}$-n | Kp: 107—108°C/2000 Pa (20 mbar) |

Die Wirksamkeit der Verbindungen der Formeln (I) bzw. (Ia) soll anhand der folgenden Beispiele erläutert werden:

Beispiel A

Plutella-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der Gewünschten

Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test z.B. die folgenden Verbindungen der Herstellungsbeispiele (Ia—1), (Ia—2), (Ia—3), (Ia—5), (Ia—6), (Ia—7) und (Ia—9) bei einer beispielhaften Wirkstoffkonzentration von 0,1% nach 3 Tagen eine Wirkung von 100%.

Beispiel B

Spodoptera-Test

Lösungsmittel: 7 Gewichtsteile Dimethylformamid
Emulgator: 1 Gwichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigten a.B. die folgenden Verbindungen der Herstellungsbeispiele (Ia—1), (Ia—2), (Ia—7) und (Ia—9) bei einer beispielhaften Wirkstoffkonzentration von 0,1% nach 3 Tagen eine Wirkung von 100%.

Beispiel C

Grenzkonzentrations-Test/Bodeninsekten

Testinsekt: Phorbia antiqua-Maden im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigten z.B. die folgenden Verbindungen der Herstellungsbeispiele (Ia—1) und (Ia—2) bei einer beispielhaften Wirkstoffkonzentration von 10 ppm eine Abtötung von 100%.

Beispiel D

Grenzkonzentrations-Test/Bodeninsekten

Testinsekt: Diabrotica balteata — Larven in Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch

genau so viele Testinsekten leben wie bei der unbehandelten Kontrolle.

Bei diesem Test zeigten z.B. die folgenden Verbindungen der Herstellungsbeispiele (Ia—1) und (Ia—2) bei einer beispielhaften Wirkstoffkonzentration von 20 ppm eine Abtötung von 100%.

Beispiel E

Grenzkonzentrations-Test

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 18°C.

Nach sechs Wochen werden die Kartoffelwurzeln auf Zysten untersucht und der Wirkungsgrad des Wirkstoffs in % bestimmt. Der Wirkungsgrad ist 100%, wenn der Befall vollständig vermieden wird, er ist 0%, wenn der Befall genau so hoch ist wie bei den Kontrollpflanzen in unbehandeltem, aber in gleicher Weise verseuchtem Boden.

Bei diesem Test zeigten z.B. die Verbindungen der Herstellungsbeispiele (Ia—1) und (Ia—2) bei einer beispielhaften Wirkstoffkonzentration von 20 ppm eine Wirkung von 100%.

Beispiel F

Fliegenmaden-Entwicklungshemmungs-Test

Testtiere: Musca domestica (multiresistent)
Lösungsmittel: Aceton

2 Gewichtsteile Wirkstoff werden in 1 000 Volumenteilen Lösungsmittel aufgenommen. Die so erhaltene Lösung wird mit weiteren Lösungsmittel auf die gewünschten Konzentrationen verdünnt.

2,5 ml Wirkstofflösung werden in eine Petrischale pipetiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 25 Testtiere in die Petrischale und bedeckt sie mit einem Glasdeckel.

14 Tage nach Ansetzen der Versuche wird die Zahl der geschlüpften Fliegen kontrolliert. Bestimmt wird der %-Satz der Schlüpfhemmung im Vergleich zur unbehandelten Kontrolle.

Bei diesem Test zeigten z.B. die folgenden Verbindungen der Herstellungsbeispiele (Ia—1) und (Ia—2) bei einer Beispielhaften Wirkstoffkonzentration von 0,02% eine Schlüpfhemmung von 100%.

Beispiel G

Test mit Boophilus microplus resistant

Lösungsmittel: 35 Gewichsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit seiben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele (Ia—1), (Ia—7), (Ia—9) und (I—3) bei einer beispielhaften Wirkstoffkonzentration von 10 000 ppm eine Wirkung von 100%.

Beispiel H

Test mit Psoroptes ovis

Lösungsmittel: 35 Gewichsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

Etwa 10—25 Psoroptes ovis werden in 1 ml der zu testenden Wirkstoffzubereitung gebracht, die in Tablettennester einer Tiefzieverpackung pipettiert wurden. Nach 24 Stunden wird der Abtötungsgrad bestimmt.

Bei diesem Test zeigten z.B. die folgenden Verbindungen der Herstellungsbeispiele (Ia—4) und (I—3) bei einer beispielhaften Wirkstoffkonzentration von 10 ppm eine Wirkung von 100%.

**Patentansprüche**

1. Schädlingsbekämpfungsmittel, gekennzeichnet durch
(a) einen Gehalt an wenigstens einem langkettigen Halogenolefin der Formel (I)

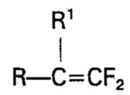

(I)

in welcher
R für einen geradkettigen oder verzweigten Alkylrest mit 8 bis 25 Kohlenstoffatomen in der geraden Kette steht, wobei der Alkylrest gegebenenfalls einfach oder mehrfach durch eine

—C≡C— und/oder 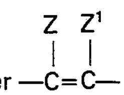 Gruppierung unterbrochen ist,

in welcher
Z und $Z^1$ gleich oder verschieden sind und für Wasserstoff oder Methyl stehen, und
$R^1$ für Wasserstoff, Alkyl oder Halogen steht, als arthropodiziden und nematiziden Wirkstoff und
(b) einen Gehalt an wenigstens einem Streckmittel oder Trägerstoff und
(c) einen Gehalt an wenigstens einem oberflächenaktiven Mittel.

2. Verfahren zur Bekämpfung von Arthropoden und Nematoden, dadurch gekennzeichet, daß man wenigstens ein eine Verbindung der Formel (I) enthaltendes Mittel gemäß Anspruch 1 auf diese Schädlinge oder ihren Lebensraum einwirken läßt.

3. Verwendung der Verbindungen der Formel (I) enthaltenden Mittel gemäß Anspruch 1 zur Bekämpfung von Arthropoden und Nematoden.

4. Verfahren zur Herstellung von Schädlingsbekämpfungsmitteln, dadurch gekennzeichnet, daß man wenigstens eine Verbindung der Formel (I) gemäß Anspruch 1 als arthropodiziden und nematiziden Wirkstoff mit Streckmitteln, Trägerstoffen und oberflächenaktiven Mitteln vermischt.

5. Halogenolefine der Formel (I)

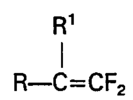

(I)

in welcher
R für einen geradkettigen oder verzweigten Alkylrest mit 8 bis 25 Kohlenstoffatomen in der geraden Kette steht, wobei der Alkylrest gegebenenfalls einfach oder mehrfach durch eine

—C≡C— und/oder 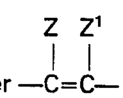 Gruppierung unterbrochen ist,

in welcher
Z und $Z^1$ gleich oder verschieden sind und für Wasserstoff oder Methyl stehen; und
$R^1$ für Wasserstoff, Alkyl oder Halogen steht,
zur Bekämpfung von Ektroparasiten bei Tieren.

**Revendications**

1. Produits pesticides caractérisés en ce que:
a) ils contiennent au moins une halogèno-oléfine à longue chaîne de formule I

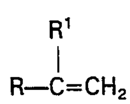

(I)

dans laquelle

R représente un groupe alkyle à chaîne droite ou ramifiée contenant 8 à 25 atomes de carbone dans la chaîne droite, ce groupe alkyle pouvant éventuellement être interrompu une ou plusieurs fois par un groupement

$$-C\equiv C-\ \text{et/ou}\ -\overset{Z}{\overset{|}{C}}=\overset{Z^1}{\overset{|}{C}}-$$

dans lequel

Z et $Z^1$, ayant des significations identiques ou différentes, représentent chacun l'hydrogène ou un groupe méthyle, et

$R^1$ représente l'hydrogène, un groupe alkyle ou un halogène, en tant que substance active arthropodicide et nématocide, et

b) ils contiennent au moins un diluant ou véhicule, et

c) ils contiennent au moins un agent tensioactif.

2. Procédé pour combattre les arthropodes et les nématodes, caractérise en ce que l'on fait agir sur ces parasites ou leur habitat des produits contenant au moins un composé de formule I selon la revendication 1.

3. Utilisation des produits contenant des composés de formule I selon la revendication 1, dans la lutte contre les arthropodes et les nématodes.

4. Procédé de préparation de produits pesticides, caractérisé en ce que l'on mélange au moins un composé de formule I selon la revendication 1, en tant que substance arthropodicide et nématocide, avec des diluants, des véhicules et des agents tensioactifs.

5. Halogéno-oléfines de formule I

$$R-\overset{R^1}{\overset{|}{C}}=CF_2 \qquad (I)$$

dans laquelle

R représente un groupe alkyle à chaîne droite ou ramifiée contenant 8 à 25 atomes de carbone dans la chaîne droite, ce groupe alkyle étant éventuellement interrompu une ou plusieurs fois par un groupement

$$-C\equiv C-\ \text{et/ou}\ -\overset{Z}{\overset{|}{C}}=\overset{Z^1}{\overset{|}{C}}$$

dans lequel

Z et $Z^1$, ayant des significations identiques ou différentes, représentent chacun l'hydrogène ou un groupe méthyle, et

$R^1$ représente l'hydrogène, un groupe alkyle ou un halogène, pour la lutte contre les ectoparasites des animaux.

**Claims**

1. Agents for combating pests, characterized in that they contain

(a) at least one long-chain halogenoolefine of the formula (I)

$$R-\overset{R^1}{\overset{|}{C}}=CF_2 \qquad (I)$$

in which

R represents a straight-chain or branched alkyl radical with 8 to 25 carbon atoms in the straight chain, the alkyl radical optionally being interrupted once or several times by a

$$-C\equiv C\ \text{and/or}\ -\overset{Z}{\overset{|}{C}}=\overset{Z^1}{\overset{|}{C}}-\ \text{grouping,}$$

in which

Z and $Z^1$ are identical or different and represent hydrogen or methyl and

$R^1$ represents hydrogen, alkyl or halogen, as an arthropodicial and nematicidal active compound, and

(b) at least one extender or carrier, and
(c) at least one surface-active agent.

2. Method of combating arthropods and nematodes, characterized in that at least one agent containing a compound of the formula (I), according to Claim 1, is allowed to act on these pests or their environment.

3. Use of agents containing compounds of the formula (I), according to Claim 1, for combating arthropods and nematodes.

4. Process for the preparation of agents for combating pests, characterized in that at least one compound of the formula (I) according to Claim 1, as an arthropodicidal and nematicidal active compound, is mixed with extenders, carriers and surface-active agents.

5. Halogenoolefines of the formula (Ia)

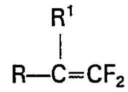

(Ia)

in which

R represents a straight-chain or branched alkyl radical with 8 to 25 carbon atoms in the straight chain, the alkyl radical optionally being interrupted once or several times by a

$-C\equiv C$ and/or 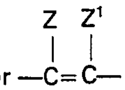 grouping,

in which

Z and $Z^1$ are identical or different and represent hydrogen or methyl, and

$R^1$ represents hydrogen, alkyl or halogen, for combating ectoparasites on animals.